# EUROPEAN PATENT SPECIFICATION

(11) **EP 1 648 476 B1**
(45) Date of publication and mention of the grant of the patent: **29.08.2007**
(21) Application number: 04743219.0
(22) Date of filing: 01.07.2004
(51) Int. Cl.: A61K 33/04, A61K 33/44, A61K 33/00, A61P 11/04, A61P 31/02

(54) **MOUTH AND/OR THROAT WASH**
MUND- UND/ODER RACHENSPÜLUNG
BAIN DE BOUCHE ET/OU GARGARISME

(30) Priority: 01.07.2003 GB 0315269
(43) Date of publication of application: 26.04.2006
(73) Proprietor: Maleno, Idalecio, Bossa, Fernandes, East Sussex TN6 2PD (GB)
(72) Inventor: Maleno, Idalecio, Bossa, Fernandes, East Sussex TN6 2PD (GB)
(74) Representative: Boden, Keith McMurray
(86) International application number: PCT/GB2004/002873
(87) International publication number: WO 2005/002598

(56) References cited:
- US-A- 5 100 653
- US-A1- 2003 003 163

## Description

This invention relates to a mouth and/or throat wash for treating *inter alia* infections of the mouth or throat.

In one aspect, the invention provides a mouth and/or throat wash which comprises an aqueous solution produced by immersing a permeable sac containing a measured quantity of a mix of potassium nitrate, activated charcoal and particulate sulphur in a measured quantity of boiling water, removing the sac from the boiling water after a predetermined period of time, and allowing the solution to cool, wherein the mix contain between 70 and 80% by weight potassium nitrate, 10 to 25% activated charcoal and between 5 and 20 % particulate sulphur.

In another aspect the invention provides a water immersible permeable sealed sac containing a measured mix of potassium nitrate, activated charcoal and particulate sulphur, the sac being usable in a treatment for infections of the mouth or throat, wherein the mix contain between 70 and 80% by weight potassium nitrate, 10 to 25% activated charcoal and between 5 and 20 % particulate sulphur..

In a further aspect, the invention provides a mouth and/or throat wash which comprises an aqueous solution produced by immersing a permeable sealed sac containing measured quantities of at least two of potassium nitrate, activated charcoal and particulate sulphur in a measured quantity of boiling water for a predetermined period of time, discarding the sac and allowing the solution to cool, wherein the mix contain between 70 and 80% by weight potassium nitrate, 10 to 25% activated charcoal and between 5 and 20 % particulate sulphur.

In a still further aspect, the invention provides a method of producing a mouth or throat wash which comprises placing a permeable sealed sac containing a measured quantity of a mix of potassium nitrate, activated charcoal and particulate sulphur in a measured quantity of water, bringing the water to the boil, removing the sac from the boiling water after a given period of time, and allowing the water containing a solution of the mix to cool.

In a still further embodiment, the invention provides a mouth and/or throat wash which comprises placing a permeable sealed sac containing a measured quantity of a mix of at least two of potassium nitrate, activated charcoal and particulate sulphur in a measured quantity of water, bringing the water to the boil, removing the sac from the boiling water after a given period of time, and allowing the water now containing a solution of the contents of the sac to cool.

By "sac" is meant a sealed bag, sachet or like container.

In one embodiment, the proportions of potassium nitrate, activated charcoal and particulate sulphur are 75:15:10.

Typically a permeable sac contains between 5 and 200g of the mix. Preferred ranges are between 30 and 150g and between 55 and 110g of the mix. The quantity of water in which the sac is immersed is typically between 50 to 750ml; preferred quantities are between 250 and 500ml and 300 and 420ml.

The predetermined period of time may be between 2 and 5 minutes. Preferably, the time period is between 3 and 4 minutes.

The sac may comprise a woven cloth of, for example, linen or like material. Other sac materials may however be used, these including paper.

In one example of a mouth or throat wash in accordance with the invention, approximately 60g of a mix of potassium nitrate, activated charcoal and particulate sulphur was placed in a permeable cloth sac. The proportions by weight of potassium nitrate, activated charcoal and particulate sulphur in the mix were 75:15:10. The open end of the sac was sealed and the sac was placed in approximately 350ml of cold water. The water was brought to the boil and the sac was left in the boiling water for approximately 4 minutes. During the boiling process, a solution of the mix diffused through the pores of the sac to create within the water a weak solution. The sac was then removed and discarded. When cooled to an acceptable temperature, the water was used as a gargle for treating a patient having a throat infection. The gargle was used when warm; preferably, the temperature of the mouthwash when taken should be as hot as is comfortable for the user. After taking 5 gargles over a time period of 15 to 30 minutes, the patient's throat infection was relieved.

In another example, a permeable cloth sac was filled with approximately 75g of potassium nitrate, activated charcoal and particulate sulphur in respective proportions by weight of 75:15:10 and the sac sealed. The sac was placed in 300ml of cold water which was brought to the boil. After 3 minutes the sac was removed from the boiling water and discarded. After cooling to an acceptable warm temperature, the water was used effectively as a mouth wash to relieve the infection after 4 gargles over a time period of around 5 minutes.

If necessary, a flavouring may be added to the mouthwash.

## Claims

1. A mouth and/or throat wash which comprises an aqueous solution produced by immersing a permeable sac containing a measured quantity of a mix of potassium nitrate, activated charcoal and particulate sulphur in a measured quantity of boiling water, removing the sac from the boiling water after a predetermined period of time, and allowing the solution to cool, wherein during the boiling process, a solution of the mix diffuses through the pores of the sac, and wherein the mix contains between 70 and 80% by weight potassium nitrate, 10 to 25% activated charcoal and between 5 and 20 % particulate sulphur.

2. A water immersible permeable sealed sac containing a measured mix of potassium nitrate, activated charcoal and particulate sulphur wherein the mix contains between 70 and 80% by weight potassium nitrate, 10 to 25% activated charcoal and between 5 and 20 % particulate sulphur, the sac being usable in a treatment for infections of the mouth or throat.

3. A method of producing a mouth or throat wash which comprises placing a permeable sealed sac containing a measured quantity of a mix of potassium nitrate, activated charcoal and particulate sulphur in a measured quantity of water, bringing the water to the boil, removing the sac from the boiling water after a given period of time, and allowing the water containing a solution of the mix to cool, wherein during the boiling process, a solution of the mix diffuses through the pores of the sac, and wherein the mix contains between 70 and 80% by weight potassium nitrate, 10 to 25% activated charcoal and between 5 and 20 % particulate sulphur.

4. A mouth wash according to claim 1, wherein proportions of potassium nitrate, activated charcoal and particulate sulphur are 75:15:10.

5. A sealed sac according to claim 2, wherein proportions of potassium nitrate, activated charcoal and particulate sulphur are 75:15:10.

6. A method according to claim 3, wherein proportions of potassium nitrate, activated charcoal and particulate sulphur are 75:15:10.

7. A sac as claimed in Claim 2 containing between 5 and 200g of the mix.

8. A sac as claimed in Claim 7 containing between 30 and 150g of the mix.

9. A sac as claimed in Claim 8 containing between 55 and 110g of the mix.

10. A method as claimed in Claim 3 wherein the sac is immersed in between 50 and 750ml of water.

11. A method as claimed in Claim 10 wherein the quantity of water is between 250 and 500ml.

12. A method as claimed in Claim 11 wherein the quantity of water is between 300 and 420ml.

13. A method as claimed in Claim 3 wherein the predetermined period of time is between 2 and 5 minutes.

14. A sac as claimed in Claim 2 produced from a woven cloth or paper.

## Patentansprüche

1. Mund- und/oder Rachenspülung, die eine wässrige Lösung umfasst, die durch Eintauchen eines durchlässigen Beutels, der eine dosierte Menge einer Mischung von Kaliumnitrat, Aktivkohle und teilchenförmigem Schwefel enthält, in eine dosierte Menge von kochendem Wasser, Entfernen des Beutels aus dem kochenden Wasser nach einer vorgegebenen Zeitperiode und Abkühlenlassen der Lösung erzeugt wird, wobei während des Kochprozesses eine Lösung der Mischung durch die Poren des Beutels diffundiert, und wobei die Mischung zwischen 70 und 80 Gewichtsprozent Kaliumnitrat, 10-25% Aktivkohle und zwischen 5 und 20% teilchenförmigen Schwefel enthält.

2. Ein in Wasser eintauchbarer durchlässiger verschlossener Beutel, der eine dosierte Mischung von Kaliumnitrat, Aktivkohle und teilchenförmigem Schwefel enthält, wobei die Mischung zwischen 70 und 80 Gewichtsprozent Kaliumnitrat, 10-25% Aktivkohle und zwischen 5 und 20% teilchenförmigen Schwefel enthält, wobei der Beutel bei der Behandlung von Infektionen des Mundes oder des Rachens brauchbar ist.

3. Verfahren zur Erzeugung einer Mund- oder Rachenspülung, die das Anordnen eines durchlässigen verschlossenen Beutels, der eine dosierte Menge einer Mischung aus Kaliumnitrat, Aktivkohle und teilchenförmigem Schwefel enthält, in einer dosierten Menge von Wasser, Bringen des Wassers zum Kochen, Entfernen des Beutels aus dem kochenden Wasser nach einer vorgegebenen Zeitperiode und Abkühlenlassen des Wassers, das eine Lösung der Mischung enthält, umfasst, wobei während des Kochprozesses eine Lösung der Mischung durch die Poren des Beutels diffundiert und wobei die Mischung zwischen 70 und 80 Gewichtsprozent 10-25% Aktivkohle und zwischen 5 und 20% teilchenförmigen Schwefel enthält.

4. Mundspülung nach Anspruch 1, bei dem die Anteile des Kaliumnitrats, der Aktivkohle und des teilchenförmigen Schwefels 75:15:10 sind.

5. Verschlossener Beutel nach Anspruch 2, bei dem die Anteile des Kaliumnitrats, der Aktivkohle und des teilchenförmigen Schwefels 75:15:10 sind.

6. Verfahren nach Anspruch 3, bei dem die Anteile des Kaliumnitrats, der Aktivkohle und des teilchenförmigen Schwefels 75:15:10 sind.

7. Beutel nach Anspruch 2, der zwischen 5 und 200 g der Mischung enthält.

8. Beutel nach Anspruch 7, der zwischen 30 und 150 g der Mischung enthält.

9. Beutel nach Anspruch 8, der zwischen 55 und 110 g der Mischung enthält.

10. Verfahren nach Anspruch 3, bei dem der Beutel in zwischen 50 und 750 ml Wasser eingetaucht wird.

11. Verfahren nach Anspruch 10, bei dem die Menge des Wassers zwischen 250 und 500 ml liegt.

12. Verfahren nach Anspruch 11, bei dem die Menge des Wassers zwischen 300 und 420 ml liegt.

13. Verfahren nach Anspruch 3, bei dem die vorgegebene Zeitperiode zwischen 2 und 5 Minuten liegt.

14. Beutel nach Anspruch 2, der aus einem Stoffgewebe oder Papier hergestellt ist.

## Revendications

1. Bain de bouche et/ou de gorge qui comprend une solution aqueuse que l'on obtient en plongeant un sac perméable contenant une quantité mesurée d'un mélange de nitrate de potassium, de charbon activé et de soufre particulaire dans une quantité mesurée d'eau en ébullition, en retirant le sac de l'eau en ébullition après un laps de temps prédéterminé, et en laissant refroidir la solution, dans lequel, au cours du processus de mise en ébullition, une solution du mélange diffuse à travers les pores du sac, et dans lequel le mélange contient du nitrate de potassium entre 70 et 80 % en poids, du charbon activé à concurrence de 10 à 25 % et du soufre particulaire entre 5 et 20 %.

2. Sac scellé perméable qui peut être plongé dans l'eau, contenant un mélange mesuré de nitrate de potassium, de charbon activé et de soufre particulaire, le mélange contenant du nitrate de potassium entre 70 et 80 % en poids, du charbon activé à concurrence de 10 à 25 % et du soufre particulaire entre 5 et 20 %, le sac étant utilisable dans un traitement pour des infections de la bouche ou de la gorge.

3. Procédé de préparation d'un bain de bouche ou de gorge, qui comprend le fait de placer un sac scellé perméable contenant une quantité mesurée d'un mélange de nitrate de potassium, de charbon activé et de soufre particulaire dans une quantité mesurée d'eau, le fait d'amener l'eau à ébullition, le fait de retirer le sac de l'eau en ébullition après un laps de temps donné, et le fait de laisser refroidir l'eau contenant une solution du mélange, procédé dans lequel, au cours du processus de mise en ébullition, une solution du mélange diffuse à travers les pores du sac, et dans lequel le mélange contient du nitrate de potassium entre 70 et 80 % en poids, du charbon activé à concurrence de 10 à 25 % et du soufre particulaire entre 5 et 20 %.

4. Bain de bouche selon la revendication 1, dans lequel les proportions du nitrate de potassium, du charbon activé et du soufre particulaire sont 75:15:10.

5. Bain de bouche selon la revendication 2, dans lequel les proportions du nitrate de potassium, du charbon activé et du soufre particulaire sont 75:15:10.

6. Bain de bouche selon la revendication 3, dans lequel les proportions du nitrate de potassium, du charbon activé et du soufre particulaire sont 75:15:10.

7. Sac selon la revendication 2, contenant entre 5 et 200 g du mélange.

8. Sac selon la revendication 7, contenant entre 30 et 150 g du mélange.

9. Sac selon la revendication 8, contenant entre 55 et 110 g du mélange.

10. Procédé selon la revendication 3, dans lequel le sac est plongé dans une quantité entre 50 et 750 ml d'eau.

11. Procédé selon la revendication 10, dans lequel la quantité d'eau se situe entre 200 et 500 ml.

12. Procédé selon la revendication 11, dans lequel la quantité d'eau se situe entre 300 et 420 ml.

13. Procédé selon la revendication 2, dans lequel le laps de temps prédéterminé se situe entre 2 et 5 minutes.

14. Sac selon la revendication 2, fabriqué en tissu ou en papier.
